(19) Europäisches Patentamt
European Patent Office
Office européen des brevets

(11) Veröffentlichungsnummer : **0 011 186**
**B1**

(12) # EUROPÄISCHE PATENTSCHRIFT

(45) Veröffentlichungstag der Patentschrift : **22.09.82**

(51) Int. Cl.³ : **C 08 F 20/20**// A61K6/08

(21) Anmeldenummer : **79104246.8**

(22) Anmeldetag : **31.10.79**

(54) **Perlpolymerisate aus viskosen Dimethacrylaten.**

(30) Priorität : **14.11.78 DE 2849280**

(43) Veröffentlichungstag der Anmeldung : **28.05.80 (Patentblatt 80/11)**

(45) Bekanntmachung des Hinweises auf die Patenterteilung : **22.09.82 Patentblatt 82/38**

(84) Benannte Vertragsstaaten : **AT CH DE FR GB IT**

(56) Entgegenhaltungen :
**FR - A - 1 005 601**
**DE - B - 1 088 716**

(73) Patentinhaber : **BAYER AG**
**Zentralbereich Patente, Marken und Lizenzen**
**D-5090 Leverkusen 1, Bayerwerk (DE)**

(72) Erfinder : **Podszun, Wolfgang, Dr.**
**Wolfskaul 4**
**D-5000 Köln 80 (DE)**
Erfinder : **Süling, Carlhans, Dr.**
**Carl-Leverkus-Strasse 10**
**D-5068 Odenthal (DE)**
Erfinder : **Walkowiak, Michael, Dr.**
**Albertus-Magnus-Strasse 10**
**D-5090 Leverkusen 1 (DE)**
Erfinder : **Schulz, Hans Hermann**
**Am Neulandkreuz 23**
**D-5653 Leichlingen (DE)**

**EP 0 011 186 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

## Perlpolymerisate aus viskosen Dimethacrylaten

Die Erfindung betrifft Polymerisate von viskosen Dimethacrylaten und gegebenenfalls bis zu 20 Gew.-% anderen Vinylmonomeren in Form von Perlen eines mittleren Durchmessers ($d_{50}$) von 10 bis 200 µm.

Derartige Perlpolymerisate aus Methacrylsäuremethylester werden als Komponente von Dentalkunststoffen, z.B. zur Herstellung von Zahnprothesen nach dem Pulver-/Flüssigkeitsverfahren gemäß DE-PS 737 058 verwendet. Sie werden durch Suspensionspolymerisation (« Perlpolymerisation ») in bekannter Weise erhalten. Bei der Verfahrensweise der Perlpolymerisation wird das Monomere in Wasser mit Hilfe eines geeigneten Dispergators unter Rühren zur gewünschten Teilchengröße zerteilt und anschließend innerhalb der einzelnen Tröpfchen mit Hilfe eines im Monomeren löslichen Initiators polymerisiert.

Für manche Zwecke, beispielsweise wenn anorganische Füllstoffe eingearbeitet werden sollen, ist es vorteilhaft, von Monomeren mit relativ hoher Viskosität auszugehen. Die Perlpolymerisation von hochviskosen Substanzen bereitet jedoch Schwierigkeiten, weil die Monomersuspensionen in verstärktem Maße zur Koagulation neigen. Es lassen sich im allgemeinen nur grobe, d.h. ein bis einige Millimeter große Perlen erhalten, deren Gestalt von der Kugelform abweicht (vgl. DE-PS 755 028 und FR-PS 1 005 601).

Es wurde nun gefunden, daß man feinteilige Perlpolymerisate (mittlerer Perlendurchmesser von 10 bis 200 µm) erhält, wenn man als Monomere bestimmte hochviskose Dimethacrylate, gegebenenfalls im Gemisch mit bis zu 20 Gew.-% anderen Monomeren, einsetzt. Diese Perlpolymerisate eignen sich insbesondere als Füllmittel in pastenförmigen Dentalmassen.

Ausgangsstoffe für die erfindungsgemäßen Perlpolymerisate sind Dimethacrylate der Formel I, worin R den Rest eines Diols darstellt, mit einer Viskosität der Dimethacrylate von 0,5 bis 500 Pa.s (gemessen bei 25 °C mit einem Rotaviskosimeter). Es eignen sich insbesondere Derivate des Bisphenol A, beispielsweise das als Bis-GMA bezeichnete Umsetzungsprodukt aus Bisphenol A und Glycidylmethacrylat der Formel II. Ebenso geeignet sind Urethan- oder Harnstoffdimethacrylate, die durch Umsetzung von Diisocyanaten mit Hydroxyalkylmethacrylaten bzw. Aminoalkylmethacrylaten gewonnen werden, beispielsweise das Reaktionsprodukt aus 2,2,4-Trimethylhexamethylendiisocyanat und 2-Hydroxyethylmethacrylat der Formel III. Es können auch Gemische verschiedener Dimethacrylate eingesetzt werden, sofern die Viskosität der Mischung im Bereich von 0,5 bis 500 Pa.s liegt, beispielsweise Mischungen aus den oben angeführten Monomeren und den Methacrylsäureestern von Ethylenglykol, Di-, Tri- oder Tetraethylenglykol.

Die viskosen Dimethacrylate oder deren ausreichend viskose Mischungen können für sich allein als Ausgangsmaterial verwendet werden, sie können aber bis zu 20 Gew.-% andere Vinylmonomere enthalten, die dann einpolymerisiert werden, beispielsweise Alkylmethacrylate wie Methylmethacrylat, Alkylacrylate wie Ethylacrylat, Styrol, Divinylbenzol oder Vinylacetat.

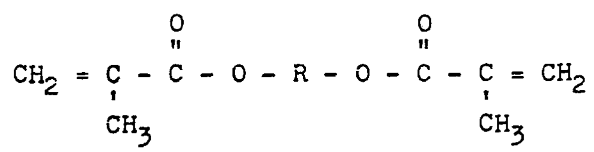
(Formel I)

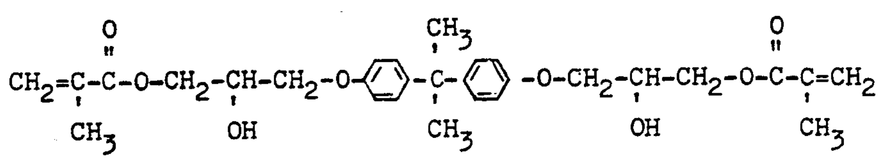
(Formel II)

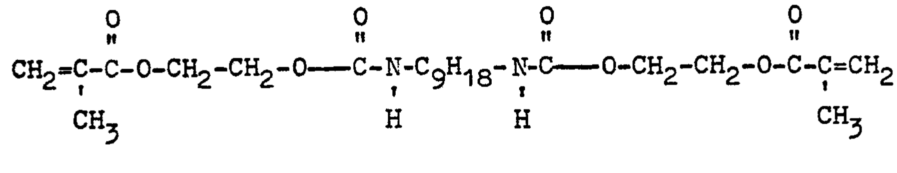
(Formel III)

Zur Herstellung der erfindungsgemäßen Polymerisatperlen wird das Monomer oder das Monomergemisch mit einer Initiatorsubstanz (z.B. Benzoylperoxid, Cyclohexylpercarbonat) versetzt und durch hochtouriges Rühren in einer wäßrigen Lösung eines hochmolekularen Dispergators suspendiert.

Als Dispergatoren eignen sich besonders gut durch partielle Verseifung gewonnene Copolymere von Vinylalkohol/Vinylazetat oder durch Copolymerisation hergestellte Methacrylsäure/Methacrylsäuremethylester-Copolymerisate.

Die erhaltene Suspension wird unter Rühren durch Erhitzen auf die Zerfallstemperatur der Initiatorsubstanz polymerisiert. Aus der auspolymerisierten Suspension kann auf bekannte Weise durch Filtrieren, Waschen und Trocknen das Perlpolymerisat gewonnen werden.

Die in den folgenden Beispielen angegebenen Viskositäten wurden mit einem Haake-Rotationsviskosimeter bei 25 °C bestimmt.

## Beispiel 1

Perlpolymerisat aus Bis-GMA

Reaktionsgefäß :
2-Liter-Planschliffbecher mit Blattrührer, Rückflußkühler, Innenthermometer, Gaseinlaß- und Gasauslaßrohr.

Mischung 1 : Monomerphase
200 g Bis-GMA
4 g Benzoylperoxid

Die Viskosität der Monomerphase beträgt 420 000 mPa.s.

Mischung 2 : wäßrige Phase
550 ml dest. Wasser
250 ml MAS MMA-Dispergatorlösung.

(7,5 %ige wäßrige Lösung eines Copolymerisats aus gleichen Gewichtsteilen Methacrylsäure und Methylmethacrylat mit pH = 6 (mit NaOH) eingestellt) und der Viskosität von 3,6 Pa.s.).

Mischung 1 und Mischung 2 werden unter Ausschluß von Luftsauerstoff in das Reaktionsgefäß gegeben und 1 Std. lang bei 50 °C mit 600 UpM gerührt. Man erhitzt die gebildete Suspension unter weiterem Rühren auf 80 °C und kühlt bei einsetzender exothermer Reaktion so stark, daß die Reaktion unter 90 °C gehalten wird. Nach dem Abklingen der Reaktion wird der Ansatz noch 2 Stunden lang auf 85 °C gehalten. Danach wird das Reaktionsgemisch mit 1 Ltr. Wasser versetzt und der pH-Wert durch Zugabe von Essigsäure auf 3 eingestellt. Das gebildete Perlpolymerisat wird durch Filtration abgetrennt, mehrfach mit dest. Wasser gewaschen und bei 80 °C getrocknet.

Ausbeute : 193 g klare, runde Perlen
mittlerer Korndurchmesser ($d_{50}$) = 100 μm.

## Beispiel 2

Perlpolymerisat aus Bis-GMA und Triethylenglykoldimethacrylat.

Reaktionsgefäß wie in Beispiel 1.

Mischung 1 : Monomerphase
188 g Bis-GMA
112 g Triethylenglykoldimethacrylat
3 g Benzoylperoxid

Die Viskosität der Monomerphase beträgt 1 200 mPa.s.

Mischung 2 : wäßrige Phase
700 ml dest. Wasser
300 ml MAS-MMA-Dispergatorlösung wie in Beispiel 1.

Mischung 1 und Mischung 2 werden 30 Min. lang bei Raumtemperatur mit 600 UpM zu einer Suspension verrührt. Die Suspension wird, wie in Beispiel 1 beschrieben, auspolymerisiert und aufgearbeitet.

Ausbeute : 290 g klare, runde Perlen
mittlerer Korndurchmesser ($d_{50}$) = 30 μm

## Beispiel 3

Perlpolymerisat aus einem Urethandimethacrylat.

Mischung 1 : Monomerphase

300 g Umsetzungsprodukt von 2,2,4-Trimethylhexamethylendiisocyanat und 2-Hydroxyethylmethacrylat

3 g Benzoylperoxid

Die Viskosität der Monomerphase beträgt 7 500 mPa.s.
Die Arbeitsweise ist wie in Beispiel 2 unter Verwendung der dort angeführten wäßrigen Phase.
Ausbeute : 276 g runde Perlen
mittlerer Korndurchmesser ($d_{50}$) = 70 μm.

## Ansprüche

1. Perlpolymerisate mit einem mittleren Perlendurchmesser von 10 bis 200 μm, hergestellt aus Dimethacrylaten der Formel

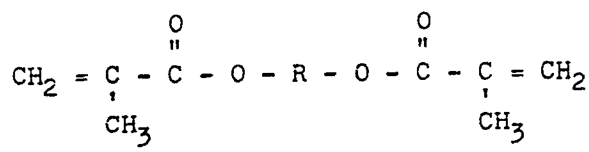
(I)

in welcher

R den Rest eines Diols darstellt, mit einer Viskosität von 0,5 bis 500 Pa.s, sowie gegebenenfalls bis zu 20 Gew.-% anderen Vinylmonomeren.

2. Perlpolymerisate nach Anspruch 1 aus dem Dimethacrylat der Formel

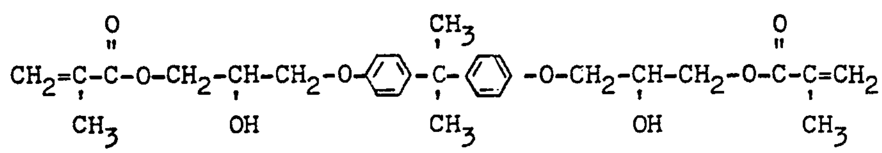
(II)

3. Perlpolymerisate nach Anspruch 1 aus dem Umsetzungsprodukt von Trimethylhexamethylendiisocyanat und 2-Hydroxyethylmethacrylat.

## Claims

1. Bead polymers with a mean bead diameter of 10 to 200 μm, produced from dimethacrylates of the formula

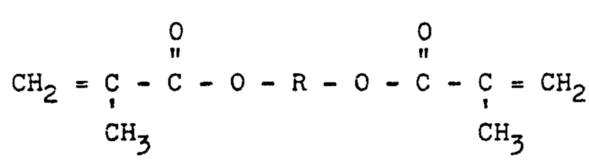
(I)

in which

R represents the residue of a diol, having a viscosity of 0.5 to 500 Pa.s, and optionally up to 20 % by weight of other vinyl monomers.

2. Bead polymers according to Claim 1 obtained from the dimethacrylate of the formula

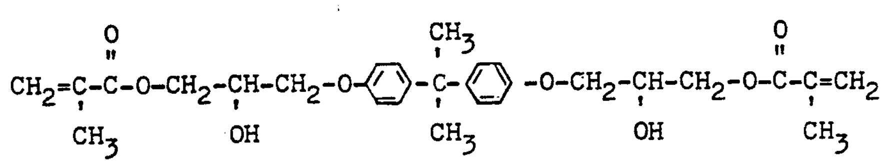
(II)

3. Bead polymers according to Claim 1 obtained from the reaction product of trimethyl hexamethylene diisocyanate and 2-hydroxyethyl methacrylate.

**Revendications**

1. Polymérisats en perles ayant un diamètre moyen des perles de 10 à 200 µm, obtenus à partir de diméthacrylates de formule :

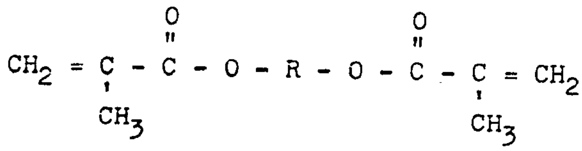

(I)

dans laquelle

R est le reste d'un diol, ayant une viscosité de 0,5 à 500 Pa.s, ainsi que, le cas échéant, jusqu'à 20 % en poids d'autres monomères vinyliques.

2. Polymérisats en perles suivant la revendication 1 du diméthacrylate de formule :

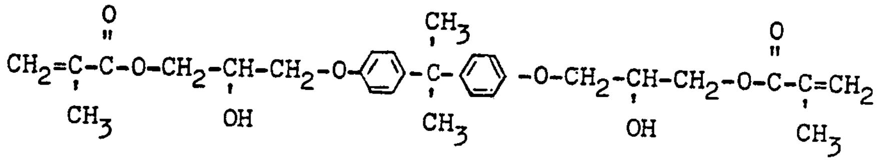

(II)

3. Polymérisats en perles suivant la revendication 1 du produit de réaction du diisocyanate de tétraméthylhexaméthylène et du méthacrylate de 2-hydroxyéthyle.